# EUROPEAN PATENT SPECIFICATION

(11) **EP 3 009 168 B1**
(45) Date of publication and mention of the grant of the patent: **16.08.2017**
(21) Application number: 14811099.2
(22) Date of filing: 06.06.2014
(51) Int. Cl.: A61Q 9/04, A61K 8/92

(54) **DEPILATORY WAX**
ENTHAARUNGSWACHS
CIRE DÉPILATOIRE

(30) Priority: 11.06.2013 ES 201330869
(43) Date of publication of application: 20.04.2016
(73) Proprietor: Laboratorios Byly S.A., 08210 Barbera del Valles (Barcelona) (ES)
(72) Inventor: ABELLO RIVAS, Rosa Maria, E-08210 Barbera del Valles (Barcelona) (ES); TORRES FERNANDEZ, Marta, E-08210 Barbera del Valles (Barcelona) (ES)
(74) Representative: Durán-Corretjer, S.L.P.
(86) International application number: PCT/ES2014/070473
(87) International publication number: WO 2014/198985

(56) References cited:
- WO-A1-2005/112876
- WO-A1-2005/112876
- WO-A2-2007/119227
- WO-A2-2008/110745
- ES-A1- 2 319 027
- FR-A- 1 256 524

## Description

The invention relates to novel compositions of depilatory waxes having improved properties in terms of better microwave oven radiation absorption and a homogeneous temperature distribution.

Depilation consists in removing superfluous hair and constitutes one of the most common hygiene habits in the female population, and is becoming increasingly popular among the male population.

There are currently various criteria for classifying the different methods of depilation. For instance, we can talk of methods requiring the aid of a specialist in their application, such as laser depilation, photodepilation or depilation by electrolysis, or methods that do not necessarily require professional intervention, such as shaving, chemical depilatories or physical depilatories. They can also be classified into: methods that cut off the hair at the level of the skin surface, such as chemical depilatories or shaving and methods that remove the hair from the root, such as physical depilatories, tweezers or electric epilators.

It should be borne in mind that to achieve the greatest performance from a depilation method, several factors need to be considered, including skin characteristics, type and amount of hair to be removed, area to be depilated, cost, convenience, durability, pain, familiarity with the depilatory technique or system to be used, and dexterity in applying the method.

The group of chemical depilatories includes depilatory creams, foams and gels, and the group of physical depilatories includes depilatory waxes.

Usually, depilation with waxes can be done cold, using strips, or hot, by applying the wax with a spatula. The latter are solid at ambient temperature and it is, therefore, necessary to heat them before application.

A type of depilatory wax that is solid at ambient temperature and lipophilic is currently available on the market, and that is heated using a microwave oven before being applied to the skin. Furthermore, once it has been applied to the skin or the area to be depilated, it can be removed without the need to use strips.

However, the main problem of the types of lipophilic depilatory waxes currently available on the market is that it is difficult for them to absorb microwave oven radiation, which can cause the microwave oven to function abnormally and/or an overheat of a part of the mass of wax, bringing with it the risk of the user suffering burns during use (Chang, AC, et al. "Depilatory Wax Burns: Experience and Investigation", Journal of Plastic Surgery, 2011, Vol. 11, 228-236).

These problems are usually solved by adding a certain amount of water to the lipophilic wax composition, although this can generate other problems such as unexpected spills due to sudden bubbling of said wax.

However, the present inventors have developed a lipophilic depilatory wax composition that does not require the addition of water, i.e. is anhydrous, which resolves the above-mentioned problems of the prior art. Surprisingly, they have discovered that better microwave oven radiation absorption and a homogeneous temperature distribution are achieved by adding glycerine to the lipophilic depilatory wax composition. This makes the depilatory wax compositions of the present invention safer for users.

More specifically, the depilatory wax composition of the present invention comprises adhesive resins, plasticisers and glycerine. Optionally, the depilatory wax composition of the present invention can comprise other components commonly used in the cosmetics sector such as emulsifiers, colourings, perfumes, proteins and derivatives, plant extracts and/or antioxidants.

An essential component in the depilatory wax of the present invention is adhesive resins. Said adhesive resins have the function of giving adhesive properties to said depilatory wax and can be of the hydrocarbon type or the rosin type and derivatives thereof. Adhesive resins suitable for use in the depilatory wax of the present invention may be selected from the group comprising esters of hydrogenated rosin and glycerine, esters of rosin and glycerine, hydrogenated styrene-methyl styrene-indene copolymers, olefin-styrene copolymers, hydrogenated polycyclopentadienes, and mixtures thereof. In the present invention, the term adhesive resins refers both to an individual adhesive resin and to a mixture of adhesive resins.

Another essential component of the depilatory wax composition of the present invention is plasticisers, the function of which is to modify the adhesion and/or cohesion of the depilatory waxes so as to ensure that said waxes cover and properly capture the hair when being applied and to allow said hair to be pulled up from the root when said waxes are removed.

Plasticisers suitable for use in the depilatory wax of the present invention can be selected from the group comprising paraffin; beeswax; ethylene vinyl acetate copolymer, alkyl methicones and olefins, microcrystalline waxes and derivatives thereof, vegetable waxes; polyethylenes; synthetic beeswaxes and dimeric alkyl esters of synthetic long-chain fatty alcohols and mixtures thereof. In the present invention, the term plasticisers refers both to an individual plasticiser and to a mixture of plasticisers.

As mentioned above, glycerine in the depilatory wax composition of the present invention has the function of improving heat absorption, and homogenising and accelerating the heating of the depilatory wax by means of a microwave oven. It can be included in the composition as a direct component or as part of some other component, such as glycerine-based plant extracts.

Optionally, the depilatory wax composition of the present invention comprises emulsifiers, the function of which is to make the various ingredients of the depilatory wax compatible, where necessary. Emulsifiers suitable for use in the depilatory wax of the present invention can be selected from the group comprising hydrogenated and ethoxylated castor oil (40 EO), hydrogenated and ethoxylated castor oil (60 EO), ethoxylated fatty alcohols and oils, nonionic, anionic, amphoteric and cationic emulsifiers; and mixtures thereof. In the present invention, the term emulsifiers refers both to an individual emulsifier and to a mixture of emulsifiers.

Furthermore, as mentioned above, the depilatory wax composition of the present invention can optionally also comprise other components commonly used in the cosmetics sector such as colourings, perfumes, proteins and derivatives, plant extracts and antioxidants.

The amount of glycerine in the depilatory wax of the present invention proves essential for obtaining the improved properties of microwave oven radiation absorption and of homogeneous temperature distribution. Said amount lies in the range 1.5 wt.% to 35 wt.% in relation to the total weight of the depilatory wax.

The quantity of adhesive resins present in the depilatory wax of the present invention lies in the range 49.5 wt.% to 83.4 wt.% in relation to the total weight of the depilatory wax. Furthermore, the quantity of plasticisers in the depilatory wax of the present invention lies in the range 10.1 wt.% to 22.6 wt.% in relation to the total weight of the depilatory wax.

An object of the present invention is, therefore, to disclose a depilatory wax having improved properties of microwave oven radiation absorption and of homogeneous temperature distribution, comprising:
a) from 49.5 wt.% to 83.4 wt.% adhesive resins;
b) from 10.1 wt.% to 22.6 wt.% plasticisers;
c) from 1.5 wt.% to 35 wt.% glycerine;

As mentioned above, the depilatory wax of the present invention can optionally comprise emulsifiers. The quantity of emulsifiers lies in the range 0.1 wt.% to 8 wt.% in relation to the total weight of the depilatory wax.

The depilatory wax of the present invention can be prepared by any mixing process known to a person skilled in the art. For example, the adhesive resins, plasticisers, glycerine and, optionally, emulsifiers can be added to the same container and the mixture heated. Subsequently, the remaining components are added, these being optional, as mentioned above. The mixture is stirred until homogeneity.

The depilatory wax of the present invention makes it possible, with the same amount of wax, the same microwave oven power and the same heating time, for the temperature of the wax containing glycerine to increase by between 14% and 29% over the reference not containing glycerine, and homogeneity of temperature throughout the mass of wax to increase by between 26% and 48% over the reference not containing glycerine.

The present invention will be described below on the basis of specific examples of depilatory wax, which must not be considered to restrict the present invention.

### EXAMPLES

### Example 1.

A depilatory wax according to the present invention was prepared, with the following composition:

| Component | Trade name | Wt.% |
|---|---|---|
| Paraffin | | 4.79 |
| Beeswax | | 9.80 |
| Ester of hydrogenated rosin and glycerine | FORALYN 9 0 | 78.25 |
| Glycerine | | 1.50 |
| Hydrogenated castor oil PEG-40 | | 0.50 |
| Hydrogenated copolymer of styrene- methyl styrene-indene | REGALITE R1010 | 4.26 |
| Pigment | | 0.50 |
| Perfume | | 0.30 |
| Plant extract | | 0.10 |
| Total | | 100.0 |

All the components were mixed in a container and heated to 120 °C, stirring until homogeneity. Subsequently, it was left to cool to room temperature (25 °C), at which the depilatory wax has a solid consistency. Then a 400 g pot of wax was placed in a microwave oven and a power of 900 was applied for 2 minutes. Homogeneous temperature distribution was achieved.

### Example 2.

A depilatory wax according to the present invention was prepared, with the following composition:

| Component | Trade name | Wt.% |
|---|---|---|
| Paraffin | | 3.33 |
| Beeswax | | 6.80 |
| Ester of hydrogenated rosin and glycerine | FORALYN 9 0 | 54.02 |
| Glycerine | | 24.00 |
| Hydrogenated castor oil PEG-40 | | 8.00 |
| Hydrogenated copolymer of styrene-methyl styrene-indene | REGALITE R1010 | 2.95 |
| Pigment | | 0.50 |
| Perfume | | 0.30 |
| Plant extract | | 0.10 |
| Total | | 100.00 |

All the components were mixed in a container and heated to 120 °C, stirring until homogeneity. Subsequently, it was left to cool to room temperature (25 °C), at which the depilatory wax has a solid consistency. Then a 400 g pot of wax was placed in a microwave oven and a power of 900 was applied for 2 minutes. Homogeneous temperature distribution was achieved.

### Example 3.

A depilatory wax according to the present invention was prepared, with the following composition:

| Component | Trade name | Wt.% |
|---|---|---|
| Paraffin | | 14.41 |
| Ethylene and vinyl acetate copolymer | ASENSA CL 300 | 8.23 |
| Hydrogenated copolymer of styrene-methyl styrene-indene | REGALITE R1090 | 71.63 |
| Glycerine | | 4.00 |
| Hydrogenated castor oil PEG-40 | | 1.33 |
| Pigment | | 0.10 |
| Perfume | | 0.30 |
| Total | | 100.0 |

The components were mixed at 120 °C, stirring until homogeneity. Subsequently, it was left to cool to room temperature (25 °C), at which the depilatory wax has a solid consistency. Then a 400 g pot of wax was placed in a microwave oven and a power of 900 was applied for 2 minutes. Homogeneous temperature distribution was achieved.

### Example 4.

A depilatory wax according to the present invention was prepared, with the following composition:

| Component | Trade name | Wt.% |
|---|---|---|
| Paraffin | | 9.89 |
| Ethylene and vinyl acetate copolymer | ASENSA CL 300 | 5.65 |
| Hydrogenated copolymer of styrene-methyl styrene-indene | REGALITE R1090 | 49.06 |
| Glycerine | | 35.00 |
| Pigment | | 0.10 |
| Perfume | | 0.30 |
| Total | | 100.00 |

The components were mixed at 120 °C, stirring until homogeneity. Subsequently, it was left to cool to room temperature (25 °C), at which the depilatory wax has a solid consistency. Then a 400 g pot of wax was placed in a microwave oven and a power of 900 was applied for 2 minutes. Homogeneous temperature distribution was achieved.

Although the invention has been described in relation to examples of preferred embodiments, these should not be considered to restrict the invention, which is to be defined by the broadest interpretation of the following claims.

## Claims

1. Anhydrous depilatory wax, **characterised in that** it comprises:
a) from 49.5 wt.% to 83.4 wt.% adhesive resins;
b) from 10.1 wt.% to 22.6 wt.% plasticisers;
c) from 1.5 wt.% to 35 wt.% glycerine;

2. Depilatory wax according to claim 1, **characterised in that** it also comprises from 0.1 wt.% to 8 wt.% emulsifiers.

3. Depilatory wax according to either claim 1 or claim 2, **characterised in that** it also comprises colourings, perfumes, proteins and derivatives, plant extracts and/or antioxidants.

4. Depilatory wax according to any of the previous claims, **characterised in that** said adhesive resins are selected from the group comprising esters of hydrogenated rosin and glycerine; esters of rosin and glycerine; hydrogenated styrene-methyl styrene-indene copolymers; olefin-styrene copolymers; hydrogenated polycyclopentadienes; and mixtures thereof.

5. Depilatory wax according to any of the previous claims, **characterised in that** said plasticisers are selected from the group comprising paraffin; beeswax; ethylene-vinyl acetate copolymer; alkyl methicones and olefins; microcrystalline waxes and derivatives thereof; vegetable waxes; polyethylenes; synthetic beeswaxes and dimeric alkyl esters of synthetic long-chain fatty alcohols; and mixtures thereof.

6. Depilatory wax according to claim 2, **characterised in that** said emulsifiers are selected from the group comprising hydrogenated and ethoxylated castor oil (40 EO), hydrogenated and ethoxylated castor oil (60 EO), ethoxylated fatty alcohols and oils, nonionic, anionic, amphoteric and cationic emulsifiers,; and mixtures thereof.

7. Use of 1.5 wt % to 35 wt % glycerine to improve microwave oven radiation absorption and for homogeneous temperature distribution and accelerated heating in anhydrous depilatory waxes.

## Patentansprüche

1. Wasserfreies Enthaarungswachs, **dadurch gekennzeichnet, dass** es das Folgende umfasst:
a) 49,5 Gew.-% bis 83,4 Gew.-% Klebharze;
b) 10,1 Gew.-% bis 22,6 Gew.-% Weichmacher;
c) 1,5 Gew.-% bis 35 Gew.-% Glycerin.

2. Enthaarungswachs nach Anspruch 1, **dadurch gekennzeichnet, dass** es auch 0,1 Gew.-% bis 8 Gew.-% Emulgiermittel umfasst.

3. Enthaarungswachs nach Anspruch 1 oder Anspruch 2, **dadurch gekennzeichnet, dass** es auch Farbstoffe, Parfüms, Proteine und Derivate, Pflanzenextrakte und/oder Antioxidationsmittel umfasst.

4. Enthaarungswachs nach einem der vorhergehenden Ansprüche, **dadurch gekennzeichnet, dass** die Klebeharze aus der Gruppe ausgewählt sind, bestehend aus hydriertem Harz und Glycerin; Estern von Harz und Glycerin; hydrierten Styrol-Methylstyrol-Inden-Copolymeren; Olefin-Styrol-Copolymeren; hydrierten Polycyclopentadienen; und Mischungen davon.

5. Enthaarungswachs nach einem der vorhergehenden Ansprüche, **dadurch gekennzeichnet, dass** die Weichmacher aus der Gruppe ausgewählt sind, umfassend Paraffin; Bienenwachs; EthylenVinylacetat-Copolymer; Alkylmethicone und Olefine; mikrokristalline Wachse und Derivate davon; pflanzliche Wachse; Polyethylene; synthetische Bienenwachse und dimere Alkylester von synthetischen langkettigen Fettalkoholen; und Mischungen davon.

6. Enthaarungswachs nach Anspruch 2, **dadurch gekennzeichnet, dass** die Emulgiermittel aus der Gruppe ausgewählt sind, umfassend hydriertes und ethoxyliertes Castoröl (40 EO), hydriertes und ethoxyliertes Castoröl (60 EO), ethoxylierte Fettalkohole und Öle, nichtionische, anionische, amphotere und kationische Emulgiermittel; und Mischungen davon.

7. Verwendung von 1,5 Gew.-% bis 35 Gew.-% Glycerin, um die Absorption von Mikrowellenofenstrahlung zu verbessern und für eine homogene Temperaturverteilung und beschleunigte Erwärmung in wasserfreien Enthaarungswachsen.

## Revendications

1. Cire dépilatoire anhydre, **caractérisée en ce qu'**elle comprend :
a) de 49.5 % en poids et 83.4 % en poids de résines adhésives ;
b) de 10.1 % en poids et 22.6 % en poids de plastifiants ;
c) de 1.5 % en poids à 35 % en poids de glycérine ;

2. Cire dépilatoire selon la revendication 1, **caractérisée en ce qu'**elle comprend également de 0.1 % en poids à 8 % en poids d'émulsifiants.

3. Cire dépilatoire selon l'une ou l'autre des revendications 1 et 2, **caractérisée en ce qu'**elle comprend également des colorants, des parfums, des protéines et leurs dérivés, des extraits de plantes et/ou des antioxydants.

4. Cire dépilatoire selon l'une quelconque des revendications précédentes, **caractérisée en ce que** lesdites résines adhésives sont choisies à partir du groupe comprenant des esters de colophane hydrogénée et de glycérine ; des esters de colophane et de glycérine ; des copolymères hydrogénés de styrène-méthyle et de styrène-indène ; des copolymères d'oléfine-styrène ; des polycyclopentadiènes hydrogénés ; et des mélanges de ceux-ci.

5. Cire dépilatoire selon l'une quelconque des revendications précédentes, **caractérisée en ce que** lesdits plastifiants sont choisis à partir du groupe comprenant la paraffine ; la cire d'abeille , le copolymère d'éthylène-vinyle acétate ; les alkyle méthicones et les oléfines ; les cires micro cristallines et les dérivés de celles-ci , les cires végétales ; les polyéthylènes ; les cires d'abeille synthétiques et les esters alkyle dimères d'alcool gras à chaîne longue synthétiques ; et les mélanges de ceux-ci.

6. Cire dépilatoire selon la revendication 2, **caractérisée en ce que** lesdits émulsifiants sont choisis à partir du groupe comprenant l'huile de castor (40 EO) hydrogénée et éthoxylée, l'huile de castor (60 EO) hydrogénée et éthoxylée, les huiles et alcool gras éthoxylés, les émulsifiants non-ioniques, anioniques, amphotériques et cationiques ; et les mélanges de ceux-ci.

7. Utilisation de 1.5 % en poids à 35 % en poids de glycérine pour améliorer l'absorption des radiations en four micro-ondes et pour une distribution de température homogène et un échauffement accéléré dans les cires dépilatoires anhydres.
